# EUROPÄISCHE PATENTSCHRIFT

(11) **EP 0 515 933 B1**
(45) Veröffentlichungstag und Bekanntmachung des Hinweises auf die Patenterteilung: **16.07.1997**
(21) Anmeldenummer: 92108269.9
(22) Anmeldetag: 15.05.1992
(51) Int. Cl.: C07D 229/00, C07C 267/00, C08G 18/79

(54) **Verfahren zur Herstellung flüssiger, lagerstabiler Carbodiimid- und/oder Uretonimingruppen aufweisender organischer Isocyanate und ihre Verwendung zur Herstellung von Polyurethankunststoffen**
Process for the preparation of fluid and storage stable organic isocyanates containing carbodiimide uretonimino groups, and their use in the preparation of polyurethane resins
Procédé pour la préparation d'isocyanates organiques liquides, stables au stockage, contenant des groupes carbodiimido uretonimino et leur utilisation pour la préparation de résines de polyuretane

(30) Priorität: 28.05.1991 DE 4117384
(43) Veröffentlichungstag der Anmeldung: 02.12.1992
(73) Patentinhaber: BAYER AG, 51368 Leverkusen (DE)
(72) Erfinder: Scholl, Hans Joachim, Dr., W-5000 Köln 80 (DE); Welte, Rainer, Dr., W-5090 Leverkusen 1 (DE)

(56) Entgegenhaltungen:
- DE-A- 2 537 685
- DE-A- 2 614 323
- ANGEWANDTE CHEMIE. INTERNATIONAL EDITION Bd. 20, Nr. 10, Oktober 1981, WEINHEIM DE Seiten 819 - 830 K. WAGNER ET AL. 'ALPHA, OMEGA-DIISOCYANAT OCARBODIIMIDES,- POLYCARBODIIMIDES, AND THEIR DERIVATIVES.'

## Beschreibung

Die Erfindung betrifft ein neues Verfahren zur Herstellung flüssiger, lagerstabiler Carbodiimid- und/oder Uretonimingruppen aufweisender Isocyanatmischungen, die nach diesem Verfahren erhältlichen Gemische und ihre Verwendung bei der Herstellung von Polyurethankunststoffen, vorzugsweise Polyurethanschaumstoffen.

Carbodiimid- und/oder Uretonimingruppen aufweisende Isocyanatmischungen sind in besonders einfacher Weise mit den hierfür bei weitem wirksamsten Katalysatoren aus der Phospholinoxid-Reihe nach der grundlegenden Verfahrensweise der US-Patentschrift 2 853 473 herzustellen.

Diese hohe Katalysefähigkeit ist einerseits sehr erwünscht, um die Carbodiimidisierungsreaktion unter schonenden Temperaturbedingungen anzustoßen, andererseits ist aber bis heute kein Verfahren bekannt, das eine wirksame Abstoppung der Phospholinoxid-Katalyse ohne Einschränkung gewährleistet. Eine derartige, wirksame Abstoppung ist insbesondere dann von großem technischen Interesse, wenn flüssige, lagerstabile Isocyanatmischungen mit bestimmten Anteilen an Carbodiimid- und/oder Uretonimingruppen gefordert sind, sei es zur Verflüssigung von 4,4'-Diisocyanatodiphenylmethan, oder zur Herstellung von lagerstabilen Polyisocyanatmischungen mit festgelegtem NCO-Gehalt, z.B. zur Herstellung Carbodiimid-haltiger Schaummaterialien.

Wegen des hohen technischen Interesses hat es naturgemäß nicht an Versuchen gefehlt, eine wirksame Abstoppung der Phospholinoxid-Katalyse zu finden:

So soll wasserfreie Salzsäure ausreichend desaktivierend wirken (Angew. Chem. 93, S. 859 (1981). In der DE-OS 2 614 323 wird Thionylchlorid als bevorzugter "Entaktivator" offenbart.

Gemäß DE-OS 2 537 685 sind diese und alle anderen Zusätze als Desaktivatoren geeignet, die mit dem Katalysator unter Addukt- oder Salzbildung reagieren. Andererseits wird in der gleichen DE-OS 2 537 685 an anderer Stelle darauf hingewiesen, daß eine derartige Desaktivierung aussichtslos erschien, "da es aus der DE-OS 2 245 634 bekannt war, daß eben solche Addukte selbst noch Carbodiimidisierungskatalysatoren für Isocyanate darstellen". Daher wird empfohlen, möglichst geringe Mengen Katalysator (ppb bis ppm-Bereich) und große Überschüsse an Desaktivator zu verwenden. Ganz abgesehen von der verunreinigenden, aktivitätsmindernden Wirkung derartiger Desaktivatormengen ist auch diese Maßnahme zur Erzielung einer langen Lagerstabilität ohne weitere, wenn auch stark verminderte CO₂-Abspaltung und damit verbundenem Viskositätsanstieg nicht ausreichend, weil dieser hochwirksame Katalysator (bzw. entsprechende Adukkte) als "echter" Katalysator auch in größter Verdünnung prohibitive Restaktivität behält. Naturgemäß bleibt diese Restaktivität umso größer, je mehr Phospholinoxid-Katalysator verwendet werden muß, um die Carbodiimidisierungsreaktion anzustoßen. Dies gilt z.B. für Polyisocyanatmischungen der Diphenylmethanreihe mit Sumpfanteilen und daraus resultiereden reaktionshemmenden Verunreinigungen.

Es war daher die der Erfindung zugrundeliegende Aufgabe ein neues Verfahren zur Herstellung flüssiger, lagerstabiler Carbodiimid- und/oder Uretonimingruppen aufweisender Isocyanatmischungen zur Verfügung zu stellen, welches die angesprochenen Mängel beheben hilft. Wie überraschenderweise gefunden wurde, konnte diese Aufgabe durch die nachstehend näher beschriebene Erfindung gelöst werden.

Gegenstand der Erfindung ist ein Verfahren zur Herstellung flüssiger, lagerstabiler Carbodiimid- und/oder Uretonimingruppen aufweisender organischer Isocyanate durch teilweise Carbodiimidisierung von Isocyanatgruppen mit Katalysatoren vom Phospholin-Typ, dadurch gekennzeichnet, daß die Carbodiimidisierungsreaktion durch den Zusatz einer silylierten Säure der Formel X-[Si(CH₃)₃]ₙ gestoppt wird, wobei
- X: für den neutralen Säurerest steht, wie er durch Entfernung der aciden Wasserstoffatome aus einer n-basischen Säure mit einem pKa-Wert von maximal 3 erhalten wird, wobei Halogenwasserstoffsäuren ausgenommen sind, und
- n: eine ganze Zahl von 1 bis 3 bedeutet.

Erfindungsgemäß bevorzugt ist,
- daß man als silylierte Säuren O-silylierte, Sauerstoff enthaltende Säuren verwendet, die in nicht silylierter Form einen pKa-Wert von maximal 2 aufweisen,
- daß man als silylierte Säuren Trifluormethansulfonsäuretrimethylsilylester oder Phosphorsäuretris(trimethylsilylester) verwendet,
- daß man als organisches Isocyanat aromatische Diisocyanate, ausgewählt aus der Gruppe bestehend aus (i) 2,4- und/oder 2,6-Diisocyanatotoluol, (ii) 2,2'- und/oder 4,4'-Diisocyanatodiphenylmethan und (iii) beliebigen Gemischen diese Diisocyanate verwendet,
- daß man als organische Isocyanate Polyisocyanatgemische der Diphenylmethanreihe mit einem Gehalt an Diisocyanatodiphenylmethan-Isomeren von 80 bis 100 Gew.-% und 0 bis 20 Gew.-% an höher als difunktionellen Polyisocyanaten der Diphenylmethanreihe verwendet, wobei sich die Diisocyanatodiphenylmethan-Isomeren zu 40 bis 80 Gew.-% aus 4,4'-Diisocyanatodiphenylmethan, 20 bis 60 Gew.-% aus 2,4'-Diisocyanatodiphenylmethan und 0 bis 8 Gew.-% aus 2,2'-Diisocyanatodiphenylmethan zusammensetzen und sich die genannten Prozentsätze zu 100 ergänzen,
- daß man als organisches Isocyanat Polyphenylpolymethylen-polyisocyanate verwendet, wie sie durch Anilin-Formaldehyd-Kondensation und anschließende Phosgenierung herstellt werden ("roh MDI").

Die Erfindung betrifft auch die flüssigen Polyisocyanatmischungen, die nach dem obengenannten Verfahren erhältlich sind.

Gegenstand der Erfindung ist schließlich auch die Verwendung der erfindungsgemäßen flüssigen Polyisocyanatmischungen zur Herstellung von Polyurethankunststoffen, vorzugsweise Polyurethanschaumstoffen mit verbessertem Brandverhalten.

Als Ausgangsmaterialien für das erfindungsgemäße Verfahren können beliebige organische Isocyanate eingesetzt werden. Vorzugsweise wird das erfindungsgemäße Verfahren jedoch zur Carbodiimidisierung von organischen Diisocyanaten der in der Polyurethanchemie eingesetzten Art verwendet.

Hierzu gehören insbesondere:
1) Aromatische Diisocyanate wie 2,4- und/oder 2,6-Diisocyanatotoluol (TDI), 2,2'-, 2,4'- und/oder 4,4'-Diisocyanatodiphenylmethan (MDI) bzw. beliebige Gemische derartiger aromatischer Diisocyanate.
2) Polyisocyanatgemische der Diphenylmethanreihe mit einem Gehalt an Diisocyanatodiphenylmethan-Isomeren von 80 bis 100 Gew.-% und 0 bis 20 Gew.-% an höher als difunktionellen Polyisocyanaten der Diphenylmethanreihe, wobei sich die Diisocyanatodiphenylmethan-Isomeren zu 40 bis 80 Gew.-% aus 4,4'-Diisocyanatodiphenylmethan, 20 bis 60 Gew.-% aus 2,4'-Diisocyanatodiphenylmethan und 0 bis 8 Gew.-% aus 2,2'-Diisocyanatodiphenylmethan zusammensetzen und sich die genannten Prozentsätze zu 100 ergänzen.
3) Polyphenylpolymethylen-polyisocyanate, wie sie durch Anilin-Formaldehyd-Kondensation und anschließende Phosgenierung hergestellt werden ("roh-MDI").

Das erfindungsgemäße Verfahren wird in Gegenwart der benannten hochwirksamen Katalysatoren aus der Phospholinreihe durchgeführt, z.B. eines handelsüblichen Gemisches der Phospholinoxide der Formeln:

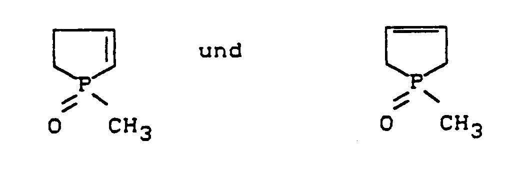

Die Menge des eingesetzten Katalysators hängt von der Qualität der Ausgangsisocyanate ab. Die jeweils notwendige Katalysatormenge läßt sich daher am einfachsten in einem Vorversuch bestimmen.

Die Carbodiimidisierungsreaktion wird gemäß Erfindung im allgemeinen im Temperaturbereich zwischen 50 bis 150°C, vorzugsweise 60 bis 100°C, durchgeführt. Die optimale Reaktionstemperatur richtet sich nach der Art der eingesetzten Ausgangsisocyanate und kann in einem einfachen Vorversuch ermittelt werden.

Die Carbodiimidisierungsreaktion wird im allgemeinen bei Erreichen eines Carbodiimidisierungsgrads (Carbodiimidisierungsgrad=Prozentsatz der carbodiimidisierten Isocyanatgruppen, bezogen auf Gesamtmenge der in Ausgangsisocyanat vorliegenden Isocyanatgruppen) von 3 bis 35, vorzusgweise 5 bis 30 %, abgebrochen. Der Carbodiimidisierungsgrad kann während des erfindungsgemäßen Verfahrens an der Menge des dem Reaktionsgemisch entweichenden Kohlendioxids erkannt werden. Diese volumetrisch bestimmbare Kohlendioxidmenge gibt somit zu jedem Zeitpunkt während des erfindungsgemäßen Verfahrens Auskunft über den erreichten Carbodiimidisierungsgrad.

Zur Beendigung der Carbodiimidisierungsreaktion wird das erfindungswesentliche Zusatzmittel als Abstopper eingesetzt.

Bei den erfindungswesentlichen Zusatzmitteln handelt es sich um silylierte Säuren der Formel

X-[Si(CH₃)₃]ₙ.

In dieser Formel haben X und n die bereits obengenannte Bedeutung. Vorzugsweise steht
- X: für den neutralen Säurerest einer n acide Wasserstoffatome aufweisenden, Sauerstoff enthaltenden Säure eines maximalen pKa-Werts von 2.

Geeignet sind beispielsweise entsprechende silylierte Sulfonsäuren wie Trifluormethansulfonsäuretrimethylsilylester oder Methansulfonsäuretrimethylsilylester, silylierte Ester von Säuren des Phosphors wie Phosphorsäuretris(trimethylsilylester) oder Phosphorsäurediethylester-trimethylsilylester.

Die beispielhaft genannten, erfindungswesentlichen Zusatzmittel werden beim erfindungsgemäßen Verfahren in Mengen eingesetzt, die mindestens der Menge des eingesetzten Katalysators äquivalent sind. Bevorzugt sind 1 bis 2 Mol Zusatzmittel pro Mol Katalysator. Die Zugabe erfolgt normalerweise bei herrschender Reaktionstemperatur; danach wird das so abgestoppte Reaktionsgemisch auf Raumtemperatur gekühlt.

Die Wirksamkeit der erfindungswesentlichen Zusatzmittel ist augenscheinlich:
a) sofortiges Abklingen der CO₂-Entwicklung nach Zusatz,
b) die erfindungsgemäßen Produkte spalten kein CO₂ bei Lagerung ab (kein Druckaufbau in verschlossenen Gefäßen), während bei Vergleichsprodukten nach dem bisherigen Stand der Technik Druckaufbau durch CO₂-Abspaltung zu beobachten ist.

Das Kriterium "Druckaufbau durch weitere CO₂-Abspaltung" ist von entscheidender Bedeutung zur Prüfung der Produktequalität bei Lagerung. Üblicherweise offenbarte Datenwerte wie Viskosität oder NCO-Gehalt sind weniger hilfreich, weil:
1a) Carbodiimid- und Isocyanatgruppen Uretonimingruppen ausbilden; 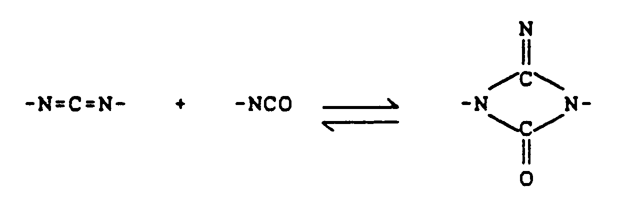 es handelt sich um eine temperaturabhängige Gleichgewichtsreaktion, die bei Raumtemperatur weitgehend auf der Seite des Uretonimins liegt. IR-Spektren frisch hergestellter erfindungsgemäßer Produkte zeigen bei Raumtemperatur-Lagerung über einen Zeitraum von mehreren Tagen den langsamen Übergang der Carbodiimidgruppierung in den Uretoniminring, so daß für diesen Zeitraum Viskositätsanstiege nicht automatisch eine Lagerinstabilität signalisieren,
1b) die übliche Bestimmung des NCO-Gehalts nicht die übliche Genauigkeit aufweisen kann, da neben den NCO-Gruppen (auch der "verkappten" NCO-Gruppe im Uretonimin) wechselnde Anteile an Carbodiimidgruppen miterfaßt werden können.

Die erfindungsgemäßen Polyisocyanatgemische können selbstverständlich in an sich bekannter Weise mit geeigneten Blockierungsmitteln für Isocyanatgruppen wie z.B. Phenol, ε-Caprolactam, Malonsäurediethylester oder Acetessigsäureethylester, blockiert werden.

Die erfindungsgemäßen Polyisocyanatgemische bzw. ihre durch die genannte Blockierungsreaktion erhaltenen Derivate stellen wertvolle Ausgangsmaterialien zur Herstellung von Polyurethankunststoffen nach dem Isocyanat-Polyadditionsverfahren dar.

So lassen sich z.B. die erfindungsgemäßen Polyisocyanatgemische aus der Diphenylmethanreihe mit einem Gehalt an höherfunktionellen Anteilen, vorzugsweise zur Herstellung von Polyurethanschaumstoffen, insbesondere zur Herstellung von im wesentlichen geschlossenzelligen Urethan-, Harnstoff-, Biuret-, Isocyanurat- und Carbodiimidgruppen aufweisenen Hartschaumstoffen und ganz besonders bevorzugt zur Herstellung von entsprechenden wassergetriebenen, FCKW-freien Hartschaumstoffen mit verbessertem Brandverhalten verwenden. Die folgenden Ausführungsbeispiele erläutern die Erfindung.

### Ausführungsbeispiele

### Beschreibung der Versuche

### A) Carbodiimidisierungsbeispiele

### Ausgangsprodukte

### 1) Katalysator:

technisches Gemisch aus 1-Methyl-1-phospha-2-cyclopenten-1-oxid und 1-Methyl-1-phospha-3-cyclopenten-1-oxid

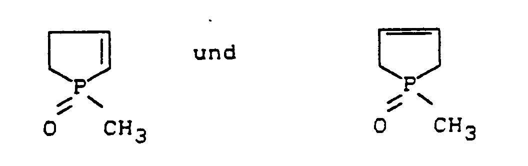

### 2) Isocyanate

| Isocyanatgemisch 1 | | |
|---|---|---|
| 56 Gew.-% | 4,4' MDI | NCO-Gehalt: 32,4 % |
| 29 Gew.-% | 2,4' MDI | |
| 5 Gew.-% | 2,2' MDI | |
| 10 Gew.-% | polymeres MDI | |

| Isocyanatgemisch 2 | | |
|---|---|---|
| 46-47 Gew.-% | 4,4' MDI | NCO-Gehalt: 33,3 % |
| 52-53 Gew.-% | 2,4' MDI | |
| <1 Gew.-% | 2,2' MDI | |

| Isocyanatgemisch 3 | | |
|---|---|---|
| 59 Gew.-% | 4,4' MDI | NCO-Gehalt: 32,2 % |
| 23 Gew.-% | 2,4' MDI | |
| 3 Gew.-% | 2,2' MDI | |
| 15 Gew.-% | polymeres MDI | |

| Isocyanatgemisch 4 | |
|---|---|
| rohes Diphenylmethandiisocyanat | NCO-Gehalt: 31,2 % |
| Viskosität 24°C: 100 mPa.s | |

| Isocyanatgemisch 5 | |
|---|---|
| rohes Diphenylmethandiisocyanat | NCO-Gehalt: 30,9 % |
| Viskosität 24°C: 200 mPa.s | |

### Beispiel 1

2,08 kg Isocyanatgemisch 1 wurden unter N₂/Rühren auf 85°C erwärmt und mit 1,4 g einer 5 %igen Katalysatorlösung (0,6 mMol) in Toluol versetzt. Nach 8 Stunden sind 29 l CO₂ entwickelt worden (Gasuhr). Danach wurde die Carbodiimidisierung durch Zugabe von 0,21 g Trifluormethansulfonsäuretrimethylsilylester (0,9 mMol) abgestoppt. Man erhielt ein lagerstabiles, flüssiges Polyisocyanatgemisch, das nach 10 Tagen Lagerung bei Raumtemperatur weitgehend Uretonimingruppen aufwies, mit den Daten:

| | |
|---|---|
| NCO: 25,5 % | Viskosität (23°C): 1000 mPas |

### Beispiel 2

2,08 kg Isocyanatgemisch 3 wurden gemäß Beispiel 1 mit 1,2 g (0,5 mMol) Katalysatorlösung 4 1/2 Stunden zur Reaktion gebracht (25 l CO₂) unt mit 2,1 g Katalysatorlösung (0,9 mMol) (10 %ig in Diisopropylether) gestoppt. Man erhielt ein flüssiges, lagerstabiles Polyisocyanatgemisch, dessen Carbodiimidgruppen nach 15 Tagen Lagerung bei Raumtemperatur weitgehend in Uretonimingruppen überführt sind.

| | |
|---|---|
| NCO : 26,1 % | Viskosität (23°C): 900 mPas |

### Vergleichsbeispiel

Beispiel 2 wurde wiederholt und mit 0,12 g Butylcarbamidsäurechlorid (0,9 mMol) gestoppt. Das Produkt entwickelte ständig CO₂, (Druckaufbau) so daß es nach 15 Tagen bei einer ansteigenden Viskosität von 1600 mPas verworfen werden mußte.

### Beispiel 3

1 kg 4,4'-Diisocyanato-diphenylmethan wurden auf 90°C erwärmt und mit 0,1 g einer 3 %igen Katalysatorlösung (0,026 mMol) in Toluol versetzt. Nach 3 Std. wurde bei einer CO₂-Entwicklung von 10,4 l mit 0,2 g einer 5 %igen Lösung von Trifluormethansulfonsäuretrimethylsilylester in Diisopropylether (0,045 mMol) gestoppt.

Man erhielt ein lagerstabiles, flüssiges Polyisocyanat, das nach 15 Tagen Lagerung bei Raumtemperatur weitgehend Uretonimingruppen aufwies, mit den Daten:

| | |
|---|---|
| NCO : 28,7 % | Viskosität: 60 mPas |

### Vergleichsbeispiel

Beispiel 1 der DE-OS 2 537 685 wurde wiederholt. Obwohl das Verhältnis Katalysator : Stopper 1:30 betrug, wies das Produkt nach 15 Tagen Lagerung vergleichsweise schlechtere Werte auf:

| | |
|---|---|
| NCO : 27,8 % | Viskosität (23°C): 90 mPas |

Nach 90 Tagen war die Viskosität auf 180 mPas angestiegen.

### Beispiel 4

1,008 kg Isocyanatgemisch 2 wurden mit 0,1 g einer 3 %igen Katalysatorlösung (0,026 mMol) 4 Stunden bei 80°C zur Reaktion gebracht (11 l CO₂) und mit 0,1 g einer 10 %igen Lösung von Trifluormethansulfonsäuretrimethylsilylester in Diisopropylether (0,045 mMol) gestoppt. Man erhielt ein lagerstabiles, flüssiges Polyisocyanatgemisch, das nach 10 Tagen Lagerung bei Raumtemperatur weitgehend Uretonimingruppen enthielt.

| | |
|---|---|
| NCO: 28 % | Viskosität (23°C): 100 mPas |

### Beispiel 5

Beispiel 1 wurde mit 0,08 g Katalysator (0,7 mMol) wiederholt. Nach 5 Stunden waren 40 l CO₂ abgespaltet. Der Ansatz wurde mit 2,08 kg Isocyanatgemisch 1 verdünnt und mit 0,4 g (1,3 mMol) Phosphorsäuretris(trimethylsilylester) gestoppt.

| | |
|---|---|
| NCO : 27,0 % | Viskosität (23°C): 400 mPas |
| (nach 10 Tagen bei Raumtemperatur) | |

### Beispiel 6

2,18 kg Isocyanatgemisch 5 wurden mit 2,5 g einer 5 %igen Katalysatorlösung (1,1 mMol) 5 Stunden bei 80°C zur Reaktion gebracht (10,3 l) und mit 4,8 g einer 10 %igen Lösung von Trifluormethansulfonsäuremethylsilylester in Diisopropyleter (2,2 mMol) gestoppt.

| | |
|---|---|
| NCO : 27,7 % | Viskosität (23°C): 1400 mPas |
| (nach 10 Tagen) | |

### Beispiel 7

2,18 kg Isocyanatgemisch 4 wurden mit 1 g einer 10 %igen Katalysatorlösung in Toluol (0,9 mMol) 3 Stunden bei 85°C zur Reaktion gebracht (10,2 l) und mit 0,3 g Trifluormethansulfonsäuretrimethylsilylester (1,3 mMol) gestoppt.

| | |
|---|---|
| NCO : 28,5 % | Viskosität (23°C): 600 mPas |
| (nach 10 Tagen) | |

### B) Verschäumungsbeispiele

Die nachfolgende Tabelle 1 listet Schaumrezepturen unter Mitverwendung des erfindungsgemäßen, flüssigen Polyisocyanatgemisches auf (Beispiel 1/Beispiel a).

Im Vergleichsbeispiel b) wird das entsprechende Ausgangs-Isocyanatgemisch 1, das keiner Carbodiimidisierungsreaktion unterworfen wurde, analog verschäumt.

Vergleichsbeispiel c) enthält als NCO-Komponente ein entsprechendes Prepolymer. Die Herstellung daraus resultierender FCKW-freier Hartschäume erfolgt in an sich bekannter Weise.

Tabelle 2 belegt das vergleichsweise deutlich verbesserte Brandverhalten des Hartschaums aus Beispiel a) unter Verwendung des erfindungsgemäßen Polyisocyanats.

**Tabelle 1**

| | Beispiel a) | Vergleichsbeispiele | |
|---|---|---|---|
| | | b) | c) |
| Weichschaumpolyether 1∗ | 60,1 | 60,1 | 60,1 |
| Hartschaumpolyester 1∗ | 12,0 | 12,0 | 12,0 |
| Hartschaumpolyether 2∗ | 12,0 | 12,0 | 12,0 |
| Flammschutzmittel | 57,1 | 57,1 | 57,1 |
| Glycerin∗ | 2,6 | 2,6 | 2,6 |
| Wasser∗ | 7,2 | 7,2 | 7,2 |
| Polyethersiloxan∗ | 3,5 | 3,5 | 3,5 |
| Katalysator 1∗ | 3,0 | 3,0 | 3,0 |
| Katalysator 2∗ | 1,9 | 1,9 | 1,9 |
| Polyisocyanat A∗ | 398,0 | - | - |
| Polyisocyanat B∗ | - | 312 | - |
| Polyisocyanat C∗ | - | - | 416 |
| NCO-Kennzahl | 200 | 200 | 200 |
| Startzeit (s) | 11 | 6 | 11 |
| Abbindezeit (s) | 55 | 34 | 59 |
| Rohdichte (kg/m³) | 33,8 | 30,1 | 38,4 |

| | | | |
|---|---|---|---|
| ∗Menge in Gew.-Teilen | | | |

- Weichschaumpolyether 1: Polyether der OH-Zahl 28 mit Propylenglykol als Starter und 87 % Propylenoxid (PO) und endständig 13 % Ethylenoxid (EO)
- Hartschaumpolyester 1: Polylester der OH-Zahl 213 aus Adipinsäure/Phthalsäure (1:0,5) und Glycerin/Propylenglykol
- Hartschaumpolyether 2: Polypropylenoxidether der OH-Zahl 865 mit Trimethylolpropan als Starter
- Flammschutzmittel: Trichlorisopropylphosphat
- Polyethersiloxan: handelsüblicher Stabilisator (B 1605 der Fa. Goldschmidt AG)
- Katalysator 1: 25 % Kaliumacetat in Diethylenglykol
- Katalysator 2: N,N-Dimethylcyclohexylamin
- Polyisocyanat A: erfindungsgemäßes Polyisocyanatgemisch nach Beispiel 1
- Polyisocyanat B: Isocyanatgemisch 1
- Polyisocyanat C: Prepolymer aus 88 % Isocyanatgemisch 1 und 12 % Polypropylenoxidether der OH-Zahl 515 mit Propylenglykol als Starter, NCO-Gehalt: 24,5 %.

**Tabelle 2**

| Prüfergebnisse Brandverhalten (nach DIN 4102) | | |
|---|---|---|
| Beispiele aus Tabelle 1 | | Kantenbeflammung |
| | Probe | Erreichen der Meßmarke 150 mm (s) |
| Beispiel a) | 1 | x |
| | 2 | x |
| | 3 | x |
| | 4 | x |
| | 5 | x |
| Beispiel b) (Vergleich) | 1 | 9 |
| | 2 | 10 |
| | 3 | 10 |
| | 4 | 9 |
| | 5 | 10 |
| Beispiel c) (Vergleich) | 1 | 7 |
| | 2 | 8 |
| | 3 | 7 |
| | 4 | 7 |
| | 5 | 7 |
| x ≙ Meßmarke nicht erreicht | | |

Die Beispiele b) und c) (Vergleichsbeispiele) bestehen den Test der Kantenbeflammung nicht. Ihr Brandverhalten ist demnach deutlich ungünstiger und als "leicht entflammbar" einzustufen.

## Patentansprüche

1. Verfahren zur Herstellung flüssiger, lagerstabiler Carbodiimid- und/oder Uretonimingruppen aufweisender organischer Isocyanate durch teilweise Carbodiimidisierung von Isocyanatgruppen mit Katalysatoren vom Phospholin-Typ, dadurch gekennzeichnet, daß die Carbodiimidisierungsreaktion durch den Zusatz einer silylierten Säure der Formel
X-[Si (CH₃)₃ ]ₙ
gestoppt wird, wobei
X für den neutralen Säurerest steht, wie er durch Entfernung der aciden Wasserstoffatome aus einer n-basischen Säure mit einem pKa-Wert von maximal 3 erhalten wird, wobei Halogenwasserstoffsäuren ausgenommen sind,und
n eine ganze Zahl von 1 bis 3 bedeutet.

2. Verfahren gemäß Anspruch 1, dadurch gekennzeichnet, daß man als silylierte Säuren O-silylierte, Sauerstoff enthaltende Säuren verwendet, die in nicht siliylierter Form einen pKa-Wert von maximal 2 aufweisen.

3. Verfahren gemäß Anspruch 1 und 2, dadurch gekennzeichnet, daß man als silylierte Säuren Trifluormethansulfonsäuretrimethylsilylester oder Phosphorsäuretris(trimethylsilylester) verwendet.

4. Verfahren gemäß Anspruch 1 bis 3, dadurch gekennzeichnet, daß man als organisches Isocyanat aromatische Diisocyanate, ausgewählt aus der Gruppe bestehend aus (i) 2,4- und/oder 2,6-Diisocyanatotoluol, (ii) 2,2'- und/oder 4,4'-Diisocyanatodiphenylmethan und (iii) beliebigen Gemischen dieser Diisocyanate verwendet.

5. Verfahren gemäß Anspruch 3, dadurch gekennzeichnet, daß man als organisches Isocyanat Polyisocyanatgemische der Diphenylmethanreihe mit einem Gehalt an Diisocyanatodiphenylmethan-Isomeren von 80 bis 100 Gew.-% und 0 bis 20 Gew.-% an höher als difunktionellen Polyisocyanaten der Diphenylmethanreihe verwendet, wobei sich die Diisocyanatodiphenylmethan-Isomeren zu 40 bis 80 Gew.-% aus 4,4'-Diisocyanatodiphenylmethan, 20 bis 60 Gew.-% aus 2,4'-Diisocyanatodiphenylmethan und 0 bis 8 Gew.-% aus 2,2'-Diisocyanatodiphenylmethan zusammensetzen und sich die genannten Prozentsätze zu 100 ergänzen.

6. Verfahren gemäß Anspruch 1 bis 3, dadurch gekennzeichnet, daß man als organisches Isocyanat Polyphenylpolymethylen-polyisocyanate, wie sie durch Anilin-Formaldehyd-Kondensation und anschließende Phosgenierung hergestellt werden ("rohes MDI") verwendet.

7. Flüssige Polyisocyanatmischungen, erhältlich nach den Verfahren gemäß Anspruch 1 bis 6.

8. Verwendung der flüssigen Polyisocyanatmischungen gemäß Anspruch 7 zur Herstellung von Polyurethankunststoffen.

9. Verwendung der flüssigen Polyisocyanatmischungen gemäß Anspruch 7 zur Herstellung von Polyurethanschaumstoffen.

## Claims

1. Process for preparing liquid, storage-stable organic isocyanates with carbodiimide and/or uretonimino groups by partial carbodiimidisation of isocyanate groups with catalysts of the phospholine type, characterised in that the carbodiimidisation reaction is terminated by the addition of a silylated acid of the formula
X-[Si (CH₃)₃ ]ₙ
wherein
X represents the neutral acid group which is obtained by removal of the acidic hydrogen atoms from an n-basic acid with a maximum pKa value of 3, wherein hydrohalide acids are excepted, and
n is an integer from 1 to 3.

2. Process according to Claim 1, characterised in that O-silylated, oxygen-containing acids which have a maximum pKa value of 2 in the non-silylated form are used as silylated acids.

3. Process according to Claims 1 and 2, characterised in that trimethylsilyl trifluoromethanesulphonate or tris-trimethylsilyl phosphate are used as silylated acids.

4. Process according to Claims 1 to 3, characterised in that aromatic diisocyanates, selected from the group consisting of (i) 2,4 and/or 2,6-diisocyanatotoluene, (ii) 2,2' and/or 4,4'-diisocyanatodiphenylmethane and (iii) any mixture of these diisocyanates, are used as organic isocyanates.

5. Process according to Claim 3, characterised in that polyisocyanate mixtures from the diphenylmethane series with a concentration of 80 to 100 wt.% of diisocyanatodiphenylmethane isomers and 0 to 20 wt.% of more than difunctional polyisocyanates from the diphenylmethane series are used as organic isocyanate, wherein 40 to 80 wt.% of the diisocyanatodiphenylmethane isomers comprises 4,4'-diisocyanatodiphenylmethane, 20 to 60 wt.% comprises 2,4'-diisocyanatodiphenylmethane and 0 to 8 wt.% comprises 2,2'-diisocyanatodiphenylmethane and the percentages mentioned add up to 100.

6. Process according to Claims 1 to 3, characterised in that polyphenylpolymethylene polyisocyanates such as are prepared by aniline/formaldehyde condensation and subsequent phosgenation ("crude MDI") are used as organic isocyanate.

7. Liquid polyisocyanate mixtures obtainable by the process in accordance with Claims 1 to 6.

8. Use of liquid polyisocyanate mixtures in accordance with Claim 7 for preparing polyurethane plastics.

9. Use of liquid polyisocyanate mixtures in accordance with Claim 7 for preparing polyurethane foams.

## Revendications

1. Procédé de préparation d'isocyanates organiques liquides, stables à conservation, contenant des groupes carbodiimide et/ou urétonimine par carbodiimidation partielle des groupes isocyanate à l'aide de catalyseurs de type phospholine, caractérisé en ce que l'on arrête la réaction de carbodiimidation par addition d'un acide silylé de formule
X-[Si(CH₃)₃]n
dans laquelle
X représente le radical d'acide neutre tel qu'obtenu par élimination des atomes d'hydrogène acides d'un acide n-basique ayant une valeur de pKa de 3 au maximum, à l'exclusion des hydracides halogénés, et
n est un nombre entier allant de 1 à 3.

2. Procédé selon la revendication 1 caractérisé en ce que l'on utilise en tant qu'acides silylés des acides contenant de l'oxygène et O-silylés qui, à l'état non silylé, ont une valeur de pKa de 2 au maximum.

3. Procédé selon les revendications 1 et 2 caractérisé en ce que l'on utilise en tant qu'acides silylés le trifluorométhanesulfonate de triméthylsilyle ou le phosphate de tris(triméthylsilyle).

4. Procédé selon les revendications 1 à 3 caractérisé en ce que l'on utilise en tant qu'isocyanates organiques des diisocyanates aromatiques choisis parmi (i) le 2,4- et/ou le 2,6-diisocyanatotoluène, (ii) le 2,2'- et/ou le 4,4'-diisocyanatodiphénylméthane, et (iii) les mélanges quelconques de ces diisocyanates.

5. Procédé selon la revendication 3 caractérisé en ce que l'on utilise en tant qu'isocyanates organiques des mélanges de polyisocyanates de la série du diphénylméthane contenant de 80 à 100 % en poids d'isomères du diisocyanatodiphénylméthane et de 0 à 20 % en poids de polyisocyanates de la série du diphénylméthane à une fonctionnalité supérieure à 2, les isomères du diisocyanatodiphénylméthane consistant eux-mêmes pour 40 à 80 % en poids en le 4,4'-diisocyanatodiphénylméthane, pour 20 à 60 % en poids en le 2,4'-isocyanatodiphénylméthane et pour 0 à 8 % en poids en le 2,2'-diisocyanatodiphénylméthane, les pourcentages indiqués se complétant à 100.

6. Procédé selon les revendications 1 à 3 caractérisé en ce que l'on utilise en tant qu'isocyanates organiques des polyphénylpolyméthylènepolyisocyanates tels qu'obtenus par condensation aniline-formaldéhyde suivie d'une phosgénation ("MDI brut").

7. Mélanges de polyisocyanates liquides obtenus par le procédé selon les revendications 1 à 6.

8. Utilisation des mélanges de polyisocyanates liquides selon la revendication 7 pour la préparation de résines synthétiques de polyuréthanes.

9. Utilisation des mélanges de polyisocyanates liquides selon la revendication 7 pour la préparation de mousses de polyuréthanes.
